(19) Europäisches Patentamt
European Patent Office
Office européen des brevets

(11) **EP 3 049 533 B1**

(12) **EUROPEAN PATENT SPECIFICATION**

(45) Date of publication and mention
of the grant of the patent:
**26.12.2018 Bulletin 2018/52**

(21) Application number: **14781106.1**

(22) Date of filing: **25.09.2014**

(51) Int Cl.:
***C12Q 1/689*** (2018.01)

(86) International application number:
**PCT/EP2014/070574**

(87) International publication number:
**WO 2015/044315 (02.04.2015 Gazette 2015/13)**

(54) **BACTERIAL SIGNATURE OF ATOPIC DERMATITIS AND USE THEREOF IN THE PREVENTION AND/OR TREATMENT OF THIS PATHOLOGY**

BAKTERIELLE SIGNATUR VON ATOPISCHER DERMATITIS UND VERWENDUNG DAVON ZUR VORBEUGUNG UND/ODER BEHANDLUNG DIESER PATHOLOGIE

SIGNATURE BACTÉRIENNE DE LA DERMATITE ATOPIQUE ET SON UTILISATION DANS LA PRÉVENTION ET/OU LE TRAITEMENT DE CETTE PATHOLOGIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2013 FR 1359249**

(43) Date of publication of application:
**03.08.2016 Bulletin 2016/31**

(73) Proprietor: **L'Oréal
75008 Paris (FR)**

(72) Inventors:
• **MARTIN, Richard
F-37210 Rochecorbon (FR)**
• **SEITE, Sophie
F-75019 Paris (FR)**

(74) Representative: **Lavoix
2, place d'Estienne d'Orves
75441 Paris Cedex 09 (FR)**

(56) References cited:
EP-A1- 2 508 605          WO-A2-2009/018447
JP-A- 2008 107 275      JP-A- 2012 177 601

• H. H. KONG ET AL: "Temporal shifts in the skin microbiome associated with disease flares and treatment in children with atopic dermatitis", GENOME RESEARCH, vol. 22, no. 5, 6 February 2012 (2012-02-06), pages 850-859, XP055101528, ISSN: 1088-9051, DOI: 10.1101/gr.131029.111 cited in the application
• I. DEKIO ET AL: "Characterization of skin microbiota in patients with atopic dermatitis and in normal subjects using 16S rRNA gene-based comprehensive analysis", JOURNAL OF MEDICAL MICROBIOLOGY, vol. 56, no. 12, 1 December 2007 (2007-12-01), pages 1675-1683, XP055101637, ISSN: 0022-2615, DOI: 10.1099/jmm.0.47268-0
• ENSHI ZHANG ET AL: "Characterization of the skin fungal microbiota in patients with atopic dermatitis and in healthy subjects", MICROBIOLOGY AND IMMUNOLOGY, vol. 55, no. 9, 12 September 2011 (2011-09-12), pages 625-632, XP055102061, ISSN: 0385-5600, DOI: 10.1111/j.1348-0421.2011.00364.x
• Thomas Werfel: "Classification, Clinical Features and Differential Diagnostics of Atopic Dermatitis" In: "Atopic Dermatitis in Childhood and Adolescence", 1 January 2011 (2011-01-01), KARGER, Basel, XP055102114, ISSN: 1662-3886 ISBN: 978-3-80-559570-4 vol. 15, pages 1-10, DOI: 10.1159/000328130, the whole document page 6; tables 2,3

EP 3 049 533 B1

• LUKE K URSELL ET AL: "The interpersonal and intrapersonal diversity of human-associated microbiota in key body sites", JOURNAL OF ALLERGY AND CLINICAL IMMUNOLOGY, ELSEVIER, AMSTERDAM, NL, vol. 129, no. 5, 14 March 2012 (2012-03-14), pages 1204-1208, XP028421191, ISSN: 0091-6749, DOI: 10.1016/J.JACI.2012.03.010 [retrieved on 2012-03-20]

• BOURRAIN MURIEL ET AL: "Balance between beneficial microflora and Staphylococcus aureus colonisation: in vivo evaluation in patients with atopic dermatitis during hydrotherapy.", EUROPEAN JOURNAL OF DERMATOLOGY : EJD 1 DEC 2013, vol. 23, no. 6, 1 December 2013 (2013-12-01), pages 786-794, XP008167398, ISSN: 1952-4013

## Description

### Field of the invention

This invention relates to the characterization of the bacterial signature associated with atopic dermatitis and the use thereof in the prevention and/or treatment of atopic dermatitis.

### Prior art

At present, more than 500 bacterial species have been detected on healthy skins, involving more than 2 million of genes. Around $10^6$ bacteria inhabit every $cm^2$ of skin. The microbiome of the skin of healthy subjects has been described as having a specificity according to 3 types of areas: moist, dry and sebaceous.

The bacterial ecosystem of the skin acts on the immune response and contributes to clinical signs of certain cutaneous imbalances, such as atopic dermatitis.

Atopic dermatitis, also called atopic eczema, is a chronic and inflammatory disease of the skin. Atopic dermatitis preferentially concerns infants and children, but also affects adolescents and adults. The number of patients with atopic dermatitis is constantly increasing.

Atopic dermatitis is characterized by cutaneous dryness, pruritus and/or eczema, these three symptoms generally appearing in succession. In fact, the first sign of atopic dermatitis is a very dry skin, followed by the appearance of erythema and edemas, then blisters and scabby and oozing lesions. Moreover, atopic dermatitis is a disease that occurs in flare-ups.

The literature indicates that lesion areas of patients with atopic dermatitis are associated with an overabundance of the *Staphylococcus* genus, in particular *Staphylococcus aureus* (Kong et al., Genome Res, 2012).

The prevention of atopic dermatitis consists in hydrating the skin in people at risk or having already experienced one or more episodes of atopic dermatitis. The treatment of atopic dermatitis consists in hydrating the skin, reducing inflammation and relieving itching. In this regard, the application of hydrating creams and the oral or topical administration of antihistamines and/or anti-inflammatory drugs, such as cortisone, are prescribed. In flare-ups, it is important to monitor the appearance of bacterial secondary infections in lesion areas scratched by the patient. Atopic dermatitis is also a cause of sleeping problems due to the significant associated itching.

The existing treatments for atopic dermatitis are not always effective and flare-ups often occur when the treatment is stopped.

The diagnosis of atopic dermatitis is generally based on a clinical examination and history taking. The diagnosis of atopic dermatitis is difficult to establish, in particular due to the highly variable forms that atopic dermatitis may take in individuals, and even throughout the life of a single individual. The existing tests are based essentially on the search for IgE or on a full allergological examination. However, atopic dermatitis does not always result, or at least not solely, from an allergic predisposition. There is therefore no real method for prognosis and/or diagnosis of atopic dermatitis, and much less a method enabling prognosis and/or diagnosis of atopic dermatitis at an early stage, such as before the appearance of clinical signs.

There is therefore a real need for methods for prognosis and/or diagnosis of atopic dermatitis, which are preferably independent of factors promoting or causing atopic dermatitis, such as an allergic predisposition, and which enable, if possible, the prognosis and/or diagnosis of atopic dermatitis at an early stage, i.e. before the appearance of clinical signs.

### Detailed description

This invention is based on the demonstration of the existence of a particular bacterial signature at lesion areas in patients with atopic dermatitis.

The inventors in fact demonstrated that the microbiome present in lesion areas of patients with atopic dermatitis has low diversity with respect to the microbiome of non-pathological areas in these same patients.

In addition, the inventors demonstrated that the treatment of lesion areas of patients with atopic dermatitis with a La Roche Posay thermal spring water-based product enables to restore, in treated lesion areas, a microbiome whose diversity is similar or even the same as that of the microbiome of non-pathological areas.

The bacterial signature may be used to prevent flare-ups, even though the clinical symptoms of atopic dermatitis are not visible.

The present invention is defined in the claims.

The application thus discloses prognosis and/or diagnosis of atopic dermatitis in an individual by analyzing the microbiome present in areas suspected of being or suspected to become lesion areas.

This invention also enables to provide a method for selecting compounds useful in the prevention and/or treatment of atopic dermatitis, based on the effect of a compound to be tested on the diversity of a given microbiome.

A first object disclosed herein thus concerns an *in vitro* method for prognosis and/or diagnosis of atopic dermatitis in an individual, said method comprising the steps consisting in:

a) measuring the diversity level and/or the diversity profile of the microbiome of a sample from an area suspected to be a lesion area or an area suspected to become a lesion area in said individual,
b) comparing the diversity level and/or the diversity profile of the microbiome measured in step a) with the diversity level and/or the diversity profile of at least one reference microbiome, and
c) deducing whether the individual has or is at risk of having atopic dermatitis.

A second object disclosed herein concerns an *in vitro* method for monitoring the response to an atopic dermatitis treatment in a patient, comprising the steps consisting in:

a) measuring the diversity level and/or the diversity profile of the microbiome of a sample from a lesion area of said individual at least at two different times during the treatment, and
b) deducing whether the patient is responding favorably to the treatment.

A third object disclosed herein concerns an *in vitro* method for monitoring the development of atopic dermatitis in a patient, comprising the steps consisting in:

a) measuring the diversity level and/or the diversity profile of the microbiome of a sample from a lesion area of said individual at least at two different times, over a period,
b) deducing whether the atopic dermatitis is developing favorably or not.

A fourth object disclosed herein is an *in vitro* method for prognosis of the duration of an atopic dermatitis treatment in a patient comprising the steps consisting in:

a) measuring the diversity level and/or the diversity profile of the microbiome of a sample from a lesion area of said individual, preferably at least at two different times over a period,
b) comparing the level(s) of diversity of the microbiome measured in step a) with the diversity level and/or the diversity profile of at least one reference microbiome, and
c) deducing the probable duration of the treatment.

A fifth object disclosed herein concerns a method for selecting a compound useful in the prevention and/or treatment of atopic dermatitis, comprising the steps consisting in:

- placing a compound to be tested in contact with bacteria of a reference microbiome,
- measuring the diversity level and/or the diversity profile of the microbiome, and
- selecting, as a compound useful in the prevention and/or treatment of atopic dermatitis, a compound capable of increasing the diversity level with respect to the diversity level of the reference microbiome and/or enabling to cause the diversity profile of the reference microbiome to evolve toward a microbiome characteristic of a non-lesion area.

Definitions

This invention relates primarily to atopic dermatitis in humans.
By the expression "in humans", it is meant males and females of any age, in particular infants, children, adolescents, adults and elderly people.
A human being will be herein referred to by the terms patient(s) or individual(s).
The term "atopic dermatitis" is described as being associated with a deficit in *stratum corneum* lipid metabolism, and in particular ceramide metabolism. This pathology appears in the form of more or less chronic xerosis affecting a large span of the body, associated with inflammatory and pruritic flare-ups in plaques.
"Atopy" is a hereditary predisposition of the immune system to prefer hypersensitivity reactions mediated by immunoglobulins E (IgE) with respect to common antigens in the diet, the outside or domestic environment.
The term "xerosis" means a drying of the skin, also called cutaneous dryness.
The term "skin" herein encompasses both the skin and the scalp, but does not comprise mucous membranes.
The terms "area", "skin area" and "cutaneous area" are herein used interchangeably.

The phrases "microbiome of a sample", "microbiome in a sample" and "microbiome from a sample" are herein used interchangeably.

By the term "lesion area", it is herein meant an area of the skin affected by atopic dermatitis.

Unless otherwise indicated, a "lesion area" refers to an untreated lesion area.

The lesion areas are in particular characterized by skin dryness, redness, blisters, scabs or combinations thereof.

The inventors have demonstrated that lesion areas are also characterized by a microbiome of low diversity, comprising predominantly bacteria of the *Staphylococcus* genus in a proportion higher than in the microbiome of non-lesion areas.

Inflammation, pruritus and intense dryness are three characteristic symptoms of an atopic dermatitis lesion area.

The terms "pruritus" and "itching" are herein used interchangeably.

By contrast with a "lesion area", the terms "non-lesion area", "non-pathological area" or "healthy area" refer to an area of the skin not affected by atopic dermatitis, and preferably not affected by any other pathology or cutaneous wound.

By "area suspected to be a lesion area", it is meant, for example, an area of the skin having at least one characteristic selected from the group consisting of inflammation, pruritus, a scabby lesion and an oozing lesion, preferably at least two of these characteristics.

By "area suspected to become a lesion area", it is meant, for example, an area having xerosis and/or a skin area having been a lesion area.

The term "microbiome" herein refers to all genomes of bacteria present at the surface of a cutaneous area of a human being.

By the expression "method for diagnosis of atopic dermatitis", it is herein meant a method enabling to determine whether an individual has atopic dermatitis.

By the expression "method for prognosis of atopic dermatitis", it is herein meant a method enabling to determine whether an individual is at risk of having atopic dermatitis.

By the expression "prevention of atopic dermatitis", it is herein meant the prophylactic or preventive treatment of atopic dermatitis, which consists in preventing the appearance of atopic dermatitis, and in particular xerosis.

By the expression "treatment of atopic dermatitis", it is herein meant the therapeutic treatment of atopic dermatitis, which consists in reducing, inhibiting, eliminating symptoms of atopic dermatitis, such as xerosis, inflammation, eczema and pruritus, the span of lesions, or the progression of atopic dermatitis, for example by delaying, spacing apart or suppressing inflammatory and pruritic flare-ups.

Diversity of the microbiome of a cutaneous area

As indicated above, a "microbiome" refers herein to all of the genomes of the bacteria present at the surface of a cutaneous area of a human being.

By "diversity level of a microbiome", it is meant the number of different genetic sequences within said microbiome (also called OTU for "Operational Taxonomic Unit").

The measurement of OTUs may be performed by any suitable method well known to a person skilled in the art, such as, for example, described in Schloss and Handelsman (Appl. Environ. Microbiol., 2005, 71:1501-1506).

By "diversity profile of a microbiome", it is meant the number and/or proportion of different divisions of bacteria present within said microbiome, the number and/or proportion of different genera of bacteria present within said microbiome, or the number and/or proportion of the different genera and species of bacteria present within said microbiome.

The terms "division" and "phylum" are herein synonymous.

Among the known divisions of bacteria, the following may be cited: *Acidobacteria, Actinobacteria,* Aquificae, *Bacteroidetes, Chlamydiae, Chlorobi, Chloroflexi, Chrysiogenetes, Cyanobacteria, Deferribacteres, Deinococcus-Thermus, Dictyoglomi, Fibrobacteres, Firmicutes, Fusobacteria, Gemmatimonadetes, Nitrospirae, Planctomycetes, Proteobacteria, Spirochaetes, Thermodesulfobacteria, Thermotogae, Verrucomicrobia.*

Typically, the microbiome present at the surface of the skin of a human being comprises or consists of the following six divisions: *Proteobacteria, Actinobacteria, Bacteroidetes, Cyanobacteria, Firmicutes* and *Fusobacteria.*

A characteristic microbiome of a lesion area of a patient with atopic dermatitis has, for example, a clearly increased ratio of bacteria of the *Staphylococcus* genus, preferably significantly increased, with respect to an adjacent non-lesion area.

A characteristic microbiome of an area of a patient at a time preceding the appearance of an atopic dermatitis lesion has, for example, an increased ratio of bacteria of the *Staphylococcus* genus, with respect to an adjacent non-lesion area.

A characteristic microbiome of a treated lesion area of a patient with atopic dermatitis has, for example, a reduced ratio of bacteria of the *Staphylococcus* genus and an increased ratio of bacteria of the *Stenotrophomonas* with respect to an untreated lesion area.

A characteristic microbiome of a non-lesion area of a patient has, for example, at least 200 different genetic sequences, preferably at least 220 different genetic sequences, for example between 240 and 260.

The diversity level of a microbiome may be measured by any suitable technique well known to a person skilled in the art, such as RNA 16S analysis or the Sanger method.

In a preferred embodiment, the diversity level and/or the diversity profile of the microbiome is measured by RNA 16S analysis.

The RNA 16S analysis comprises the identification of the number of different genetic sequences. The RNA 16S analysis may also comprise the identification of the number of different genetic sequences corresponding to a given bacterial phylum, a given bacterial genus and/or a given bacterial species, and preferably the relative proportion of said phylum and/or said genus and/or said species within the microbiome.

In the methods according to the invention described below, reference is often made to a reference microbiome.

A reference microbiome is, for example, a microbiome characteristic of a lesion area of a patient with atopic dermatitis, a skin area at a time preceding the appearance of an atopic dermatitis lesion, a treated lesion area of a patient with atopic dermatitis or a non-lesion area.

A reference microbiome may also be a medium comprising different bacteria in various proportions that does or does not correspond to a microbiome existing in nature.

The reference microbiome may be obtained from a sample of a cutaneous area of an individual or by preparing a medium comprising the different bacteria in proportions corresponding to said reference microbiome.

By "medium", it is meant a medium suitable for the growth of bacteria. It may be a solid or liquid medium.

The diversity level and/or the diversity profile of a characteristic reference microbiome of a cutaneous area is preferably a mean of the microbiome diversity levels and/or diversity profile measured on samples from said skin area in several individuals.

By "increase the diversity of the microbiome", it is meant increasing the number of different genetic sequences within said microbiome.

The diversity level and/or diversity profile of the microbiome is, for example, measured in a sample from a skin area having a surface area of 0.2 cm$^2$ to 3 cm$^2$, preferably 0.5 cm$^2$ to 2 cm$^2$.

A sample from a given skin area is, for example, obtained by applying, on said skin area, an adhesive disc, for example a disc of the type D-Squame, or by scratching the surface of said skin area.

The area of the sample has a surface of 0.2 cm$^2$ to 3 cm$^2$, preferably 0.5 cm$^2$ to 2 cm$^2$.

In a preferred embodiment, the non-lesion area is a healthy area that is as close as possible to the lesion area.

A non-lesion area is thus preferably located near the lesion area, for example at a distance of at least 0.2 cm, at least 0.4 cm, at least 0.6 cm, at least 0.8 cm or at least 1 cm from the edges of the lesion area, and preferably at a distance of at most 3 cm or at most 2 cm from the edges of the lesion area.

The sample is preferably obtained from a skin area located at the popliteal fossa.

*In vitro* method for prognosis and/or diagnosis disclosed herein

This application thus discloses an *in vitro* method for prognosis and/or diagnosis of atopic dermatitis in an individual, said method comprising the steps consisting in:

a) measuring the diversity level and/or the diversity profile of a microbiome of a sample from an area suspected to be a lesion area or suspected to become a lesion area in said individual,
b) comparing the diversity level and/or the diversity profile of the microbiome measured in step a) with the diversity level and/or the diversity profile of at least one reference microbiome, and
c) deducing whether the patient has or is at risk of having atopic dermatitis.

The sample, the cutaneous areas, the diversity level, the diversity profile of the microbiome, and the reference microbiome are in particular as defined above.

The application specifically discloses the above-mentioned *in vitro* method for prognosis and/or diagnosis, wherein the reference microbiome is chosen from the group consisting of a microbiome characteristic of a lesion area, a microbiome characteristic of a skin area at a time preceding the appearance of an atopic dermatitis lesion, a microbiome characteristic of a treated lesion area and a microbiome characteristic of a non-lesion area.

The diversity level and/or diversity profile of the reference microbiome characteristic of a non-lesion area may be the level or a mean of diversity levels and/or diversity profile of the microbiome of a non-lesion area of said individual, or the level or a mean of diversity levels and/or diversity profile of the microbiome of one or more individuals not suffering from atopic dermatitis.

The diversity level and/or diversity profile of the reference microbiome characteristic of a non-lesion area is, for example, greater than or equal to 200 different genetic sequences, preferably greater than or equal to 230 different genetic sequences, more preferentially greater than or equal to 240 different genetic sequences.

In step c), it is deduced that the patient has or is at risk of having atopic dermatitis:

- if the diversity level of the microbiome measured in step a) is lower than the diversity level of a reference microbiome characteristic of a non-lesion area, preferably if the diversity level measured in step a) is below 200 different genetic sequences, and/or
- if the diversity level of the microbiome measured in step a) is close to the diversity level of a reference microbiome characteristic of a lesion area, and/or
- if the diversity level of the microbiome measured in step a) is close to the diversity level of a reference microbiome characteristic of a skin area at a time preceding the appearance of an atopic dermatitis lesion, and/or
- if the diversity level of the microbiome measured in step a) is comprised between the diversity level of a reference microbiome characteristic of a lesion area and that of a skin area at a time preceding the appearance of an atopic dermatitis lesion, and/or
- if the diversity level of the microbiome measured in step a) is lower than or equal to the diversity level of a reference microbiome characteristic of a skin area at a time preceding the appearance of an atopic dermatitis lesion, and/or
- if the diversity level of the microbiome measured in step a) is lower than or equal to the diversity level of a reference microbiome characteristic of a lesion area.

"A diversity level is close to a value" means, for example, that the diversity level is equal to said value, + or - 20 different genetic sequences, preferably + or - 10 different genetic sequences.

*In vitro* method for monitoring the response to an atopic dermatitis treatment

This invention thus also relates to an *in vitro* method for monitoring the response to an atopic dermatitis treatment in a patient, comprising the steps consisting in:

a) measuring the diversity level and/or the diversity profile of a microbiome of a sample from a lesion area of said individual at least at two different times during the treatment, and
b) deducing whether the patient responds favorably to the treatment.

The patient responds favorably to the treatment if the diversity level of the microbiome has increased between the two successive times and/or if the diversity profile gets closer to the diversity profile of the microbiome of a non-lesion area.

The patient does not respond favorably to the treatment if the diversity level of the microbiome has decreased between the two successive times and/or if the diversity profile gets closer to the diversity profile of the microbiome of a lesion area or a skin area at a time preceding the appearance of an atopic dermatitis lesion.

It is possible to perform measurements at a plurality of times during the treatment, at regular or non-regular intervals of time, during the entire treatment period, or only at the beginning, middle and/or end of the treatment.

It is possible for the patient to respond favorably to the treatment for a certain time, and then to no longer respond favorably to the treatment. In this case, it may be appropriate to modify or change the treatment.

*In vitro* method for monitoring the development of atopic dermatitis

This invention also relates to an *in vitro* method for monitoring the development of atopic dermatitis in a patient comprising the steps consisting in:

a) measuring the diversity level and/or the diversity profile of a microbiome of a sample from a lesion area of said individual at least at two different times over a period, and
b) deducing therefrom whether the atopic dermatitis is developing favorably or not.

The atopic dermatitis develops favorably if the diversity level of the microbiome has increased between the two successive times and/or if the diversity profile gets closer to the diversity profile of the microbiome of a non-lesion area.

The atopic dermatitis develops unfavorably if the diversity level of the microbiome has decreased between the two successive times and/or if the diversity profile gets closer to the diversity profile of the microbiome of a lesion area or a skin area at a time preceding the appearance of an atopic dermatitis lesion.

When atopic dermatitis develops unfavorably, it may be appropriate to propose an atopic dermatitis treatment if it has not been treated, or to modify or change the treatment.

*In vitro* method for prognosis of the duration of a treatment of atopic dermatitis disclosed herein

The application also discloses an *in vitro* method for prognosis of the duration of an atopic dermatitis treatment in a patient comprising the steps consisting in:

> a) measuring the diversity level and/or the diversity profile of a microbiome of a sample from a lesion area of said individual, preferably at least at two different times over a period,
> b) comparing the diversity level(s) of the microbiome measured in step a) with the diversity level and/or the diversity profile of at least one reference microbiome, and
> c) deducing therefrom the probable treatment duration.

In step b), the diversity level(s) measured in step a) may be compared with the diversity level and/or the diversity profile of at least one reference microbiome, preferably chosen from a microbiome characteristic of a lesion area, a microbiome characteristic of a skin area at a time preceding the appearance of an atopic dermatitis lesion, a microbiome characteristic of a treated lesion area and a microbiome characteristic of a non-lesion area.

Depending on whether the diversity level(s) and/or the diversity profile(s) measured in step a) more or less quickly approach the diversity level and/or diversity profile of a microbiome characteristic of a non-lesion area and/or a treated lesion area, or remain more or less close to the diversity level and/or diversity profile of a microbiome characteristic of a lesion area or a skin area at a time preceding the appearance of an atopic dermatitis lesion, it is possible to deduce the probable, more or less long, duration of the treatment.

In an advantageous disclosed embodiment, the *in vitro* method for prognosis of the duration of an atopic dermatitis treatment preferably comprises a step of producing one or more reference curves indicating the diversity level and/or the diversity profile of a microbiome at a lesion area over time, preferably indicating the values before, during and at the end of the treatment.

The reference curves are, for example, curves characteristic of the change in diversity of the microbiome in patients responding favorably to treatment, moderately favorably to treatment and/or passably to treatment.

Thus, in step b), the value(s) measured in step a) are preferably compared with one or more reference curve(s), and the probable treatment duration is deduced therefrom.

Method for selecting a compound useful in the prevention and/or treatment of atopic dermatitis

This invention also relates to a method for selecting a compound useful in the prevention and/or treatment of atopic dermatitis, comprising the steps consisting in:

- placing a compound to be tested in contact with bacteria of a reference microbiome,
- measuring the diversity level and/or diversity profile of the microbiome, and
- selecting, as a compound useful in the prevention and/or treatment of atopic dermatitis, a compound capable of increasing the diversity level with respect to the diversity level of the reference microbiome and/or enabling the diversity profile of the reference microbiome to be changed toward a microbiome characteristic of a non-lesion area.

The compound to be tested may, for example, be a La Roche Posay thermal spring water extract, a bacterium or a mixture of bacteria, an extract of one or more bacteria, a culture medium of one or more bacteria, an extract from a culture medium of one or more bacteria, or combinations thereof.

The bacteria are preferably bacteria predominant after treatment of a lesion area.

Said selection method is an *in vitro* or *ex vivo* method.

This invention particularly relates to the selection method as defined above, characterized in that the reference microbiome is characteristic of a lesion area of a patient with atopic dermatitis or an area of a patient at a time preceding the appearance of an atopic dermatitis lesion.

The reference microbiome may also be a medium comprising different bacteria in different proportions, which does not necessarily correspond to a natural microbiome.

The reference microbiome may be obtained from a sample of an individual or by preparing a medium comprising the different bacteria in proportions corresponding to said reference microbiome.

The term "medium" refers to a liquid medium.

The selection method is performed under conditions enabling the growth of bacteria in the absence of said compound to be tested.

Dermatological composition

This invention also relates to a dermatological composition comprising, as an active ingredient, at least one compound useful in the prevention and/or treatment of atopic dermatitis, for example as obtained by the selection method defined above.

In a preferred dermatological composition according to the invention, said compound useful in the prevention and/or treatment of atopic dermatitis is provided in the form of La Roche Posay thermal spring water and/or an extract of La Roche Posay thermal spring water.

This invention relates in particular to a dermatological composition as defined above, characterized in that it comprises 1% to 80% of La Roche Posay thermal spring water, preferably 20% to 60%, more preferentially 30% to 50%, the percentages being expressed in g for 100 g of composition.

The dermatological composition according to the invention is preferably suitable for topical application.

The terms "topical" and "on the skin" are herein synonymous.

The dermatological composition can thus be in any form suitable for topical use, such as a cream, a balm, a gel, an oil, a water, a pomade, a paste or a spray.

Method for preventing and/or treating atopic dermatitis

This invention also relates to a compound useful in the prevention and/or treatment of atopic dermatitis, selected according to the selection method as defined above, for use in the prevention and/or treatment of atopic dermatitis.

In a preferred embodiment, said compound is formulated in a dermatological composition.

The dermatological composition is in particular as defined above.

The dermatological composition is typically applied one to three times per day, for example two times per day, on the lesion areas and preferably also around the lesion areas, and/or on areas suspected to become lesion areas.

The treatment concerns patients with atopic dermatitis.

The prevention concerns individuals at risk of having atopic dermatitis, such as individuals having had at least one episode of atopic dermatitis in their life or having significant cutaneous dryness.

This invention relates in particular to a compound selected according to the selection method of the invention, for use in the prevention and/or treatment of atopic dermatitis in individuals diagnosed as having atopic dermatitis, or at risk of having atopic dermatitis, in particular after implementation of the prognosis and/or diagnosis method according to the invention defined above.

The treatment is performed at least until the symptoms disappear.

The treatment is preferably continued for several days or several weeks after the disappearance of the symptoms, possibly with a gradual reduction in the frequency of administration of the dermatological composition.

The efficacy of the atopic dermatitis treatment may be evaluated by the *in vitro* method for monitoring the response to an atopic dermatitis treatment as defined above.

The probable duration of the atopic dermatitis treatment may be evaluated by the *in vitro* method for prognosis of the duration of an atopic dermatitis treatment as defined above.

The application also discloses a method for preventing and/or treating atopic dermatitis comprising the administration of a dermatological composition disclosed herein on the skin, at lesion areas and preferably also around lesion areas, and/or at areas suspected to become lesion areas.

The application also discloses a compound selected according to the selection method as defined above, for use in the prevention and/or treatment of atopic dermatitis, characterized in that said compound enables to increase the diversity of the microbiome present at the surface of the skin, preferably at a lesion area of the skin or an area suspected to become a lesion area, in an individual with atopic dermatitis or at risk of having atopic dermatitis.

The application also discloses a method intended to increase the diversity of the microbiome present at the surface of the skin, preferably at a lesion area of the skin or an area suspected to become a lesion area, comprising the topical administration of at least one compound selected according to the selection method as defined above and/or a dermatological composition as defined above, in an individual with atopic dermatitis or at risk of having atopic dermatitis.

Other features and advantages of the invention will become clear from the following examples, provided for illustrative and non-limiting purposes.

**Description of the figures**

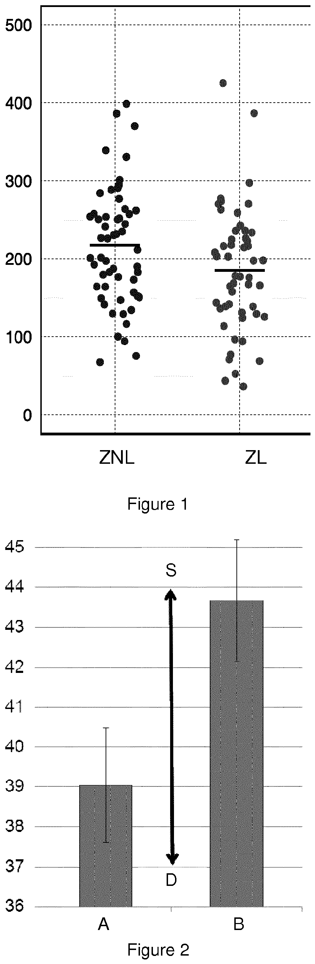

Figure 1: Diversity of the microbiome at non-lesion areas and lesion areas of patients with atopic dermatitis. The y-axis shows the number of different genetic sequences. ZNL: non-lesion area, ZL: lesion area, p<0.001.

Figure 2: Modifications in microbial diversity associated with the treatment during treatment. The y-axis shows the mean similarity of the intra-individual community before treatment and after treatment. S: toward more similarity, D: toward more dissimilarity, ("paired t-test", t=2,8303, p=0.007).

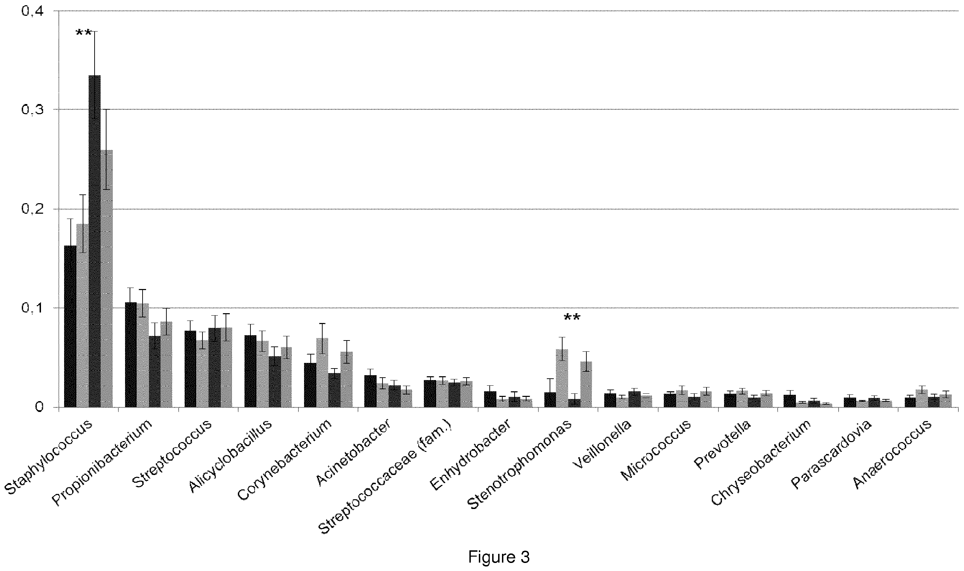

Figure 3: Mean relative abundance by genus of bacterium at lesion and non-lesion areas in the same individual with atopic dermatitis, before treatment and after treatment. For each genus, the 1st column corresponds to the results on the non-lesion area before treatment, the 2nd column corresponds to the non-lesion area after treatment, the 3rd column corresponds to the lesion area before treatment and the 4th column corresponds to the lesion area after treatment.

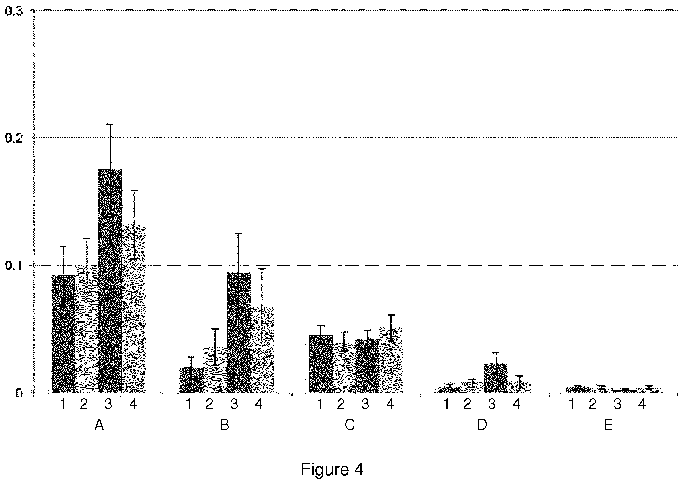

Figure 4: Mapping of the *Staphylococcus* genus by species at lesion and non-lesion areas in the same individual with atopic dermatitis, before treatment and after treatment. On the y-axis is the mean relative abundance (1 represents 100%). A: *Staphylococcus epidermidis,* B: *Staphylococcus aureus,* C: *Staphylococcus spp.,* D: *Staphylococcus haemolyticus,* E: other *Staphylococcaceae,* 1: non-lesion area before treatment, 2: non-lesion area after treatment, 3: lesion area before treatment, 4: lesion area after treatment.

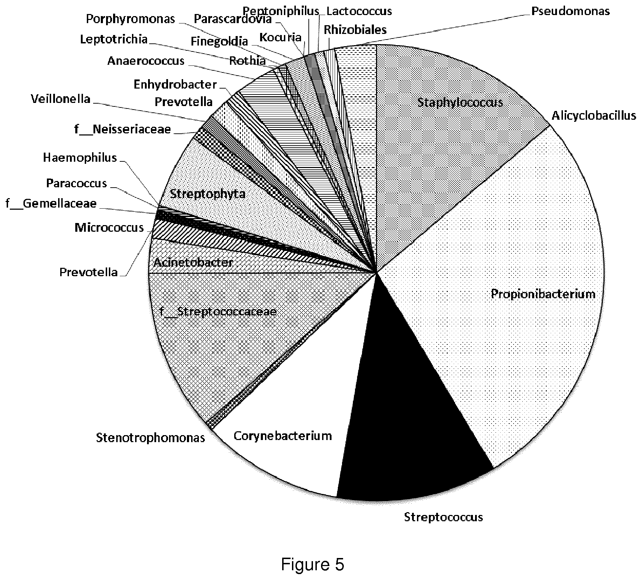

Figure 5: Photograph of the bacterial diversity by genus on non-atopic skin (mean obtained on arms of 93 non-atopic females).

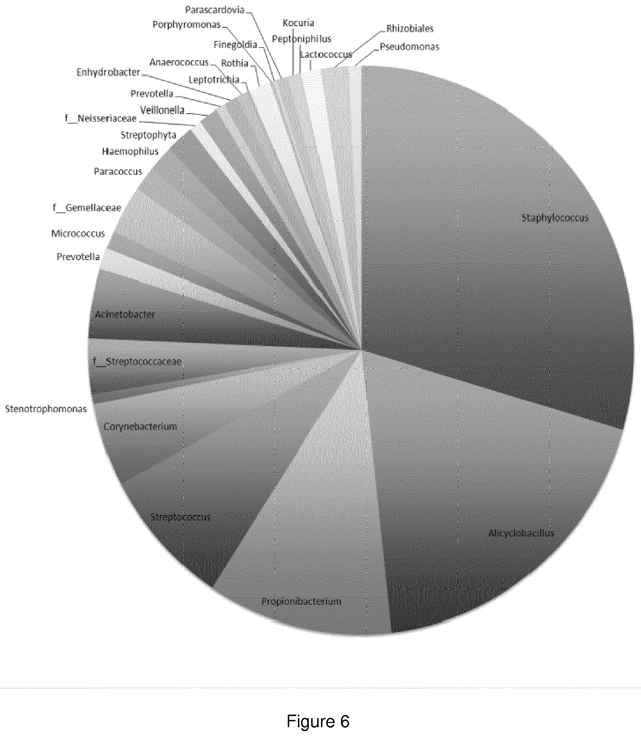

Figure 6: Photograph of the bacterial diversity by genus before the start of the treatment on lesion areas of patients with atopic dermatitis and responding favorably to treatment.

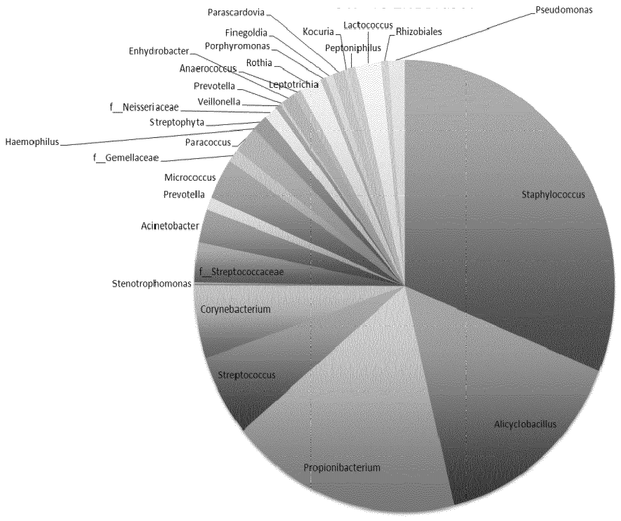

Figure 7: Photograph of the bacterial diversity by genus before the start of the treatment on non-lesion areas of patients with atopic dermatitis and responding favorably to treatment.

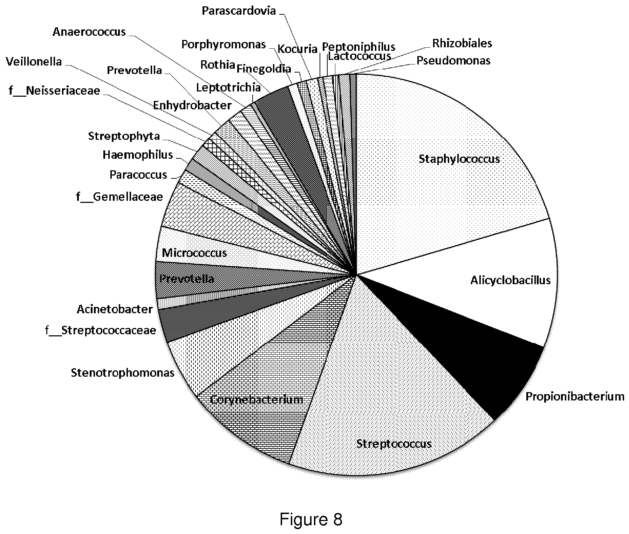

Figure 8: Photograph of the bacterial diversity by genus at 84 days after the start of treatment on lesion areas of patients with atopic dermatitis and responding favorably to treatment.

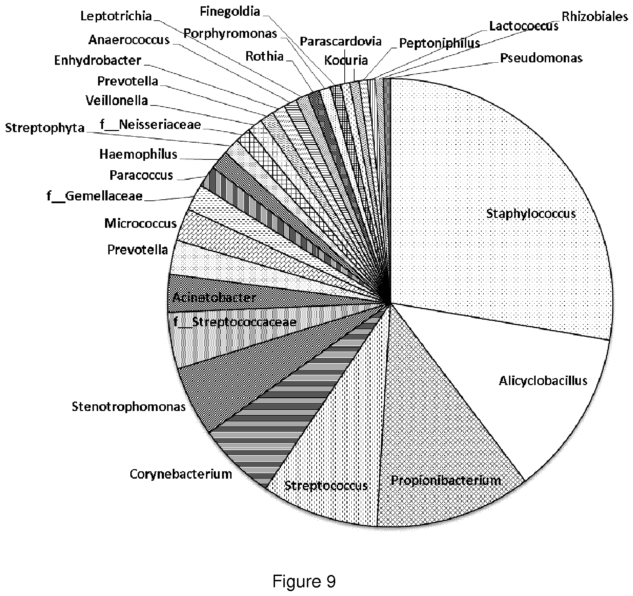

Figure 9: Photograph of the bacterial diversity by genus at 84 days after the start of treatment on non-lesion areas of patients with atopic dermatitis and responding favorably to treatment.

## Example:

### Material and methods

Patients

The study was conducted on 50 patients with atopic dermatitis and having lesion areas and non-lesion areas. The mean SCORAD (*overall severity score of atopic dermatitis*) at inclusion was $33 \pm 5.6$. The mean age was $12 \pm 9$ years.

Procedure

Les 50 patients applied the La Roche Posay thermal spring water-based product "Baume Lipikar AP" of La Roche Posay for 84 days, at a frequency of two applications per day.

Several bacterial samples are taken on lesion areas and neighboring non-lesion areas of each subject, in the period ranging from before the start of treatment until the end of treatment.

Photographs of the sampled areas are taken.

The dryness, erythema and desquamation of the areas sampled are evaluated.

The size of the sample area is small, in particular from 0.5 to 2 cm$^2$. The area sampled is the most representative of the lesion area.

The sampling is performed with sterile single-use cotton swabs under a sterile air flow. The sterile air flow is produced by a portable laminar flow hood. It filters the ambient air and enables a so-called "axenic" sample, i.e. without microorganisms from the environment. The sampler never cuts off the laminar air flow during the sampling. The sampling is performed in a quiet location and both the sampled subject and the sampler are silent during the operation. The sterile cotton swab is immersed in a milliQ water solution containing 0.15 M NaCl and 0.1% Tween 20. This solution is filtered to 0.22 $\mu$m (Minisart ref. 16534) extemporaneously under the laminar flow hood so as to ensure its sterility. The sample was standardized on the basis of the area sampled (force, time and surface). The cotton swab is broken in an Eppendorf tube sterilized at 121°C/30' guaranteed to be free of RNAse and DNAse. The samples are immediately placed at -20°C for several hours, then transferred to -80°C(preferably directly placed at -80°C) where they are stable for at least 6 months. After amplification of the bacterial DNA coding for the RNA 16S, both the bacterium genus and species are determined, by analysis using the Qiime bacterial database.

The comparison of the microbiome is performed on a lesion and non-lesion area of the same subject. The diversity of the microbiome is analyzed by several methods, comprising the Shannon Index, which is the one most commonly used by the person skilled in the art. The Shannon Index is obtained by the following formula:

$$H' = -\sum_{i=1}^{S} p_i \log_2 p_i$$

H': Shannon biodiversity index

$i$: a species from the study medium

$p_i$: Proportion of a species $i$ with respect to the total number of species (S) in the study medium (or specific richness of the medium), which is calculated as follows:

$$p(i) = n_i/N$$

where $n_i$ is the number of individuals for the species $i$ and N is the total number (the individuals of all of the species).

## Results

Clinical analysis (n=50 subjects)

At inclusion of the subjects, the overall severity score of atopic dermatitis (SCORAD) is not correlated with age or the sex of the individuals.

The age of the disease is strongly correlated with the age of the patient, which confirms the "innate" nature of the pathology (including a genetic component demonstrated with filaggrin polymorphisms) with some subjects, however, recently and severely affected (eczema acquired or cumulative with triggering environmental factors).

The severity of the erythema and dryness of the lesions sampled at inclusion are factors well correlated (erythema p=3.10-9, dryness p=1.10-4) with the overall score of the patient.

In this study, the treatment reduces the overall atopy score more considerably in males than in females.

36 patients responded favorably to the treatment and 14 patients did not respond to the treatment, comprising one patient whose SCORAD worsened during the period.

Cutaneous microflora analysis

The results concerning the number of different genetic sequences are presented in the table below:

| | | Lesion area at T0 | Non-lesion area at To | Lesion area at T84 | Non-lesion area at T84 |
|---|---|---|---|---|---|
| **Non-responders** | Number of different genetic sequences | 86 | 94 | 72 | 101 |
| | Mean number of readings per patient | 903 | 643 | 435 | 885 |
| **Responders** | Number of different genetic sequences | 119 | 121 | 118 | 138 |
| | Mean number of readings per patient | 871 | 844 | 880 | 1292 |

It should be noted that on a non-atopic arm, i.e. in an individual not suffering from atopic dermatitis, a mean of 250 different genetic sequences is detected (in an average of 92 individuals).

The patients who responded favorably, "responders", to the treatment have a greater bacterial diversity than the patients not having responded to the treatment, "non-responders".

Whether at a lesion area or a non-lesion area, the diversity is clearly lower than on non-atopic skin.

Before treatment, the microbiome of the lesion areas is significantly different from the adjacent non-lesion areas (p= 0.001).

The microbiome has less bacterial diversity than that of the adjacent areas, richness measured by two reference indices (Shannon, p=0.002 and Bray-Curtis).

The diversity tends to disappear in favor of a common signature on lesion areas, independently of the area sampled.

After a treatment, the microbiome of the lesion areas evolves into a bacterial community similar to that of the adjacent non-lesion areas (p=0.16). This microbial community remains different from that observed before treatment, regardless of the areas (p=0.002). In other words, the microbiome of the non-lesion areas at inclusion is also modified as a result of the treatment.

Staphylococci are the most represented genus of microflora in the lesion area and also in the adjacent non-lesion area (from 15 to 35% of the total microflora measured).

A significant reduction in staphylococci is observed after treatment (p<0.01), though without reaching the level observed in non-lesion areas.

More specifically, these results confirm that the species *S. aureus* as well as S. *epidermidis* are overrepresented in the lesion areas. Surprisingly, the species S. *haemoliticus* is also overabundant in the lesion areas. This overabundance of S. *haemoliticus* is fully corrected after the treatment.

The *S. epidermidis* species remains the one most highly represented in the genus before or after treatment.

The treatment of the lesion area shows a significant enrichment in the *Stenotrophomonas* genus with respect to non-treated lesion areas.

**Claims**

1. *In vitro* method for diagnosis of atopic dermatitis in an individual, said method comprising the steps consisting in:

   a) measuring the diversity level and/or the diversity profile of the microbiome of a sample from an area suspected to be a lesion area or an area suspected to become a lesion area in said individual,
   b) comparing the diversity level and/or the diversity profile of the microbiome measured in step a) with the diversity level and/or the diversity profile of at least one reference microbiome, and
   c) deducing whether the individual has atopic dermatitis.

2. Method according to claim 1, wherein the diversity level and/or the diversity profile of the microbiome is measured by RNA 16S analysis.

3. Method according to claim 1 or 2, wherein the diversity level and/or the diversity profile of the microbiome is measured in a sample from a skin area having a surface area of 0.2 cm$^2$ to 3 cm$^2$, preferably 0.5 cm$^2$ to 2 cm$^2$.

4. Method according to any one of claims 1 to 3, wherein the reference microbiome is chosen from the group consisting of a microbiome characteristic of a lesion area, a microbiome characteristic of a skin area at a time preceding the appearance of an atopic dermatitis lesion, a microbiome characteristic of a treated lesion area and a microbiome characteristic of a non-lesion area.

5. Method according to any one of claims 1 to 4, wherein the area suspected to be a lesion are is an area having at least one characteristic selected from the group consisting of an inflammation, a pruritus, a scabby lesion and an oozing lesion.

6. Method according to any one of claims 1 to 4, wherein the area suspected to become a lesion area is an area having xerosis and/or a skin area having been a lesion area.

7. *In vitro* method for monitoring the response to an atopic dermatitis treatment in a patient, comprising the steps consisting in:

   a) measuring the diversity level and/or the diversity profile of the microbiome of a sample from a lesion area of said individual at least at two different times during the treatment, and
   b) deducing therefrom whether the patient is responding favorably to the treatment, wherein the patient responds favorably to the treatment if the diversity level of the microbiome has increased between the two successive times and/or if the diversity profile gets closer to the diversity profile of the microbiome of a non-lesion area, and wherein the patient does not respond favorably to the treatment if the diversity level of the microbiome has decreased between the two successive times and/or if the diversity profile gets closer to the diversity profile of the microbiome of a lesion area or a skin area at a time preceding the appearance of an atopic dermatitis lesion.

8. *In vitro* method for monitoring the development of atopic dermatitis in a patient comprising the steps consisting in:

   a) measuring the diversity level and/or the diversity profile of the microbiome of a sample from a lesion area of said individual at least at two different times over a period, and

   b) deducing therefrom whether the atopic dermatitis is developing favorably or not, wherein the atopic dermatitis develops favorably if the diversity level of the microbiome has increased between the two successive times and/or if the diversity profile gets closer to the diversity profile of the microbiome of a non-lesion area, and the atopic dermatitis develops unfavorably if the diversity level of the microbiome has decreased between the two successive times and/or if the diversity profile gets closer to the diversity profile of the microbiome of a lesion area or a skin area at a time preceding the appearance of an atopic dermatitis lesion.

9. *In vitro* method for selecting a compound useful in the prevention and/or treatment of atopic dermatitis, comprising the steps consisting in:

   - placing a compound to be tested in contact with bacteria of a reference microbiome,
   - measuring the diversity level and/or the diversity profile of the microbiome, and
   - selecting, as a compound useful in the prevention and/or treatment of atopic dermatitis, a compound capable of increasing the diversity level with respect to the diversity level of the reference microbiome and/or enabling the diversity profile of the reference microbiome to be changed toward a microbiome characteristic of a non-lesion area.

10. Selection method according to claim 9, **characterized in that** the reference microbiome is characteristic of a lesion area of a patient with atopic dermatitis or of an area of a patient at a time preceding the appearance of an atopic dermatitis lesion.

**Patentansprüche**

1. *In vitro* Verfahren zur Diagnose von atopischer Dermatitis in einem Individuum, wobei das Verfahren die Schritte umfasst, bestehend aus:

   a) Messen des Diversitätslevels und/oder des Diversitätsprofils des Mikrobioms einer Probe aus einem Bereich, der bei dem Individuum in Verdacht steht ein Läsionsbereich zu sein oder ein Bereich der in Verdacht steht ein Läsionsbereich zu werden,

   b) Vergleichen des Diversitätslevels und/oder des Diversitätsprofils des Mikrobioms, das in Schritt a) gemessen wurde, mit dem Diversitätslevel und/oder dem Diversitätsprofil von mindestens einem Referenz-Mikrobiom, und

   c) Ableiten, ob das Individuum atopische Dermatitis aufweist.

2. Verfahren gemäß Anspruch 1, wobei das Diversitätslevel und/oder das Diversitätsprofil des Mikrobioms durch 16S-RNA-Analyse gemessen wird.

3. Verfahren gemäß Anspruch 1 oder 2, wobei das Diversitätslevel und/oder das Diversitätsprofil des Mikrobioms in einer Probe eines Hautbereichs gemessen wird, der ein Oberfläche von 0,2 $cm^2$ bis 3 $cm^2$, bevorzugt 0,5 $cm^2$ bis 2 $cm^2$ aufweist.

4. Verfahren gemäß einem der Ansprüche 1 bis 3, wobei das Referenz-Mikrobiom ausgewählt ist aus der Gruppe bestehend aus einem Mikrobiom, das charakteristisch für einen Läsionsbereich ist, einem Mikrobiom, das für einen Hautbereich zu einem Zeitpunkt vor dem Auftreten einer atopischen Dermatitisläsion charakteristisch ist, einem Mikrobiom, das für einen behandelten Läsionsbereich charakteristisch ist und einem Mikrobiom, das für einen Nicht-Läsionsbereich charakteristisch ist.

5. Verfahren gemäß einem der Ansprüche 1 bis 4, wobei der Bereich, der in Verdacht steht eine Läsion zu sein, in einem Bereich ist, der mindestens eine Eigenschaft aufweist, ausgewählt aus der Gruppe bestehend aus einer Entzündung, einer Pruritus, einer verschorften Läsion und einer nässenden Läsion.

6. Verfahren gemäß einem der Ansprüche 1 bis 4, wobei der Bereich, der in Verdacht steht ein Läsionsbereich zu werden, ein Bereich ist, der Xerosis aufweist und/oder einen Hautbereich aufweist, der ein Läsionsbereich war.

7.   *In vitro* Verfahren zum Überwachen der Reaktion auf eine Behandlung von atopischer Dermatitis bei einem Patienten, umfassend die Schritte bestehend aus:

a) Messen des Diversitätslevels und/oder des Diversitätsprofils des Mikrobioms einer Probe aus einem Läsionsbereich des Individuums mindestens an zwei verschiedenen Zeitpunkten während der Behandlung, und
b) davon Ableiten, ob der Patient günstig auf die Behandlung reagiert, wobei der Patient günstig auf die Behandlung reagiert, wenn das Diversitätslevel des Mikrobioms zwischen den zwei aufeinanderfolgenden Zeitpunkten ansteigt und/oder wenn sich das Diversitätsprofil an das Diversitätsprofil des Mikrobioms eines Nicht-Läsionsbereichs annähert und wobei der Patient ungünstig auf die Behandlung reagiert, wenn das Diversitätslevel des Mikrobioms zwischen den zwei aufeinanderfolgenden Zeitpunkten abnimmt und/oder wenn sich das Diversitätsprofil an das Diversitätsprofil des Mikrobioms eines Läsionsbereichs oder eines Hautbereichs zu einem Zeitpunkt vor dem Auftreten einer atopischen Dermatitis-Läsion annähert.

8.   *In vitro* Verfahren zum Überwachen der Entwicklung von atopischer Dermatitis bei einem Patienten, umfassend die Schritte bestehend aus:

a) Messen des Diversitätslevels und/oder des Diversitätsprofils des Mikrobioms einer Probe aus einem Läsionsbereich des Individuums mindestens an zwei verschiedenen Zeitpunkten über einen Zeitraum, und
b) davon Ableiten, ob sich die atopische Dermatitis günstig entwickelt oder nicht,
wobei sich die atopische Dermatitis günstig entwickelt, wenn das Diversitätslevel des Mikrobioms zwischen den zwei aufeinanderfolgenden Zeitpunkten ansteigt und/oder wenn sich das Diversitätsprofil an das Diversitätsprofil des Mikrobioms eines Nicht-Läsionsbereichs annähert, und wobei sich die atopische Dermatitis ungünstig entwickelt, wenn das Diversitätslevel des Mikrobioms zwischen den zwei aufeinanderfolgenden Zeitpunkten abnimmt und/oder wenn sich das Diversitätsprofil an das Diversitätsprofil des Mikrobioms eines Läsionsbereichs oder einem Hautbereich zu einem Zeitpunkt vor dem Auftreten einer atopischen Dermatitis-Läsion annähert.

9.   *In vitro* Verfahren zum Auswählen einer Verbindung, die zur Prävention und/oder Behandlung von atopischer Dermatitis geeignet ist, umfassend die Schritte bestehend aus:

- Inkontaktbringen einer zu testenden Verbindung mit Bakterien eines Referenzmikrobioms,
- Messen des Diversitätslevels und/oder des Diversitätsprofils des Mikrobioms, und
- Auswählen einer Verbindung als eine Verbindung, die zur Prävention und/oder Behandlung von atopischer Dermatitis geeignet ist, als eine Verbindung, die fähig ist das Diversitätslevel im Hinblick auf das Diversitätslevel des Referenz-Mikrobioms zu erhöhen und/oder dem Diversitätsprofil des Referenz-Mikrobioms zu ermöglichen, sich zu einem Mikrobiom zu ändern, das charakteristisch für einen Nicht-Läsionsbereich ist.

10.  Auswahlverfahren gemäß Anspruch 9, **dadurch gekennzeichnet, dass** das Referenz-Mikrobiom charakteristisch für einen Läsionsbereich eines Patienten mit atopischer Dermatitis ist oder für einen Bereich eines Patienten zu einem Zeitpunkt vor dem Auftreten einer atopischen Dermatitis-Läsion.

**Revendications**

1.   Méthode *in vitro* de diagnostic de la dermatite atopique chez un individu, ladite méthode comprenant les étapes consistant à :

a) mesurer le niveau de diversité et/ou le profil de diversité du microbiome d'un échantillon provenant d'une zone suspectée d'être une zone lésée ou une zone suspectée de devenir une zone lésée chez ledit individu,
b) comparer le niveau de diversité et/ou le profil de diversité du microbiome mesuré à l'étape a) avec le niveau de diversité et/ou le profil de diversité d'au moins un microbiome de référence, et
c) en déduire si l'individu est atteint d'une dermatite atopique.

2.   Méthode selon la revendication 1, dans laquelle le niveau de diversité et/ou le profil de diversité du microbiome est mesuré par analyse de l'ARN 16S.

3.   Méthode selon la revendication 1 ou 2, dans laquelle le niveau de diversité et/ou le profil de diversité du microbiome est mesuré dans un échantillon provenant d'une zone de peau ayant une surface de 0,2 cm$^2$ à 3 cm$^2$, de préférence

de 0,5 cm$^2$ à 2 cm$^2$.

**4.** Méthode selon l'une des revendications 1 à 3, dans laquelle le microbiome de référence est choisi dans le groupe consistant en un microbiome caractéristique d'une zone lésée, un microbiome caractéristique d'une zone de peau à un moment précédent l'apparition d'une lésion de dermatite atopique, un microbiome caractéristique d'une zone lésée traitée et un microbiome caractéristique d'une zone non lésée.

**5.** Méthode selon l'une des revendications 1 à 4, dans laquelle la zone suspectée d'être une zone lésée est une zone ayant au moins une caractéristique sélectionnée dans le groupe consistant en une inflammation, un prurit, une lésion croûteuse et une lésion suintante.

**6.** Méthode selon l'une des revendications 1 à 4, dans laquelle la zone suspectée de devenir une zone lésée est une zone présentant une xérose et/ou une zone de peau ayant été une zone lésée.

**7.** Méthode *in vitro* de suivi de la réponse à un traitement de la dermatite atopique chez un patient comprenant les étapes consistant à :

a) mesurer le niveau de diversité et/ou le profil de diversité du microbiome d'un échantillon provenant d'une zone lésée dudit individu à au moins deux instants différents au cours du traitement, et
b) en déduire si le patient répond favorablement au traitement,
ledit patient répondant favorablement au traitement si le niveau de diversité du microbiome a augmenté entre les deux instants successifs et/ou si le profil de diversité se rapproche du profil de diversité du microbiome d'une zone non lésée, et ledit patient ne répondant pas favorablement au traitement si le niveau de diversité du microbiome a diminué entre les deux instants successifs et/ou si le profil de diversité se rapproche du profil de diversité du microbiome d'une zone lésée ou d'une zone de peau à un moment précédent l'apparition d'une lésion de dermatite atopique.

**8.** Méthode *in vitro* de suivi de l'évolution d'une dermatite atopique chez un patient comprenant les étapes consistant à :

a) mesurer le niveau de diversité et/ou le profil de diversité du microbiome d'un échantillon provenant d'une zone lésée dudit individu à au moins deux instants différents au cours du temps, et
b) en déduire si la dermatite atopique évolue favorablement ou non,
la dermatite atopique évoluant favorablement si le niveau de diversité du microbiome a augmenté entre les deux instants successifs et/ou si le profil de diversité se rapproche du profil de diversité du microbiome d'une zone non lésée, et la dermatite atopique évoluant défavorablement si le niveau de diversité du microbiome a diminué entre les deux instants successifs et/ou si le profil de diversité se rapproche du profil de diversité du microbiome d'une zone lésée ou d'une zone de peau à un moment précédent l'apparition d'une lésion de dermatite atopique.

**9.** Méthode *in vitro* de sélection d'un composé utile dans la prévention et/ou le traitement de la dermatite atopique, comprenant les étapes consistant à :

- mettre un composé à tester en contact avec des bactéries d'un microbiome de référence,
- mesurer le niveau de diversité et/ou le profil de diversité du microbiome, et
- sélectionner en tant que composé utile dans la prévention et/ou le traitement de la dermatite atopique, un composé capable d'augmenter le niveau de diversité par rapport au niveau de diversité du microbiome de référence et/ou permettant de faire évoluer le profil de diversité du microbiome de référence vers un microbiome caractéristique d'une zone non lésée.

**10.** Méthode de sélection selon la revendication 9, **caractérisée en ce que** le microbiome de référence est caractéristique d'une zone lésée d'un patient atteint de dermatite atopique ou d'une zone d'un patient à un moment précédent l'apparition d'une lésion de dermatite atopique.

Figure 1

Figure 2

Figure 3

Figure 4

Figure 5

Figure 6

Figure 8

Figure 9

**EP 3 049 533 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KONG et al.** *Genome Res,* 2012

- **SCHLOSS ; HANDELSMAN.** *Appl. Environ. Microbiol.,* 2005, vol. 71, 1501-1506